# EUROPEAN PATENT APPLICATION

(11) **EP 4 122 907 A1**
(43) Date of publication of application: **25.01.2023**
(21) Application number: 21461572.6
(22) Date of filing: 23.07.2021
(51) Int. Cl.: C07C 29/80, C07C 29/76, C07C 29/74, C07C 29/88, C07C 31/22, C11C 3/00

(54) **GLYCERIN PURIFICATION METHOD AND GLYCERIN PURIFIED BY THE METHOD**

(71) Applicant: ORLEN Poludnie S.A., 32-540 Trzebinia (PL)
(72) Inventor: Borówka, Grzegorz, 28-136 Nowy Korczyn (PL); Semerjak, Grzegorz, 32-065 Krzeszowice (PL); Krzak, Mateusz, 34-122 Nidek (PL); Krasodomski, Wojciech, 31-419 Kraków (PL); Lubowicz, Jan, 31-444 Kraków (PL)
(74) Representative: Witek, Rafal

(57) **Abstract**

The first subject of the invention is a method of purifying glycerine resulting from transesterification reaction of used cooking oils or vegetable oils, comprising the steps of: removing the non-glycerol organic fraction and methanol, removing water, removing sulphur-containing impurities, glycerine distillation, removing impurities containing nitrogen, chlorine and sulphur, deodorization and bleaching, characterized in that in step a) the feed is acidified with sulphuric acid (VI) to a pH of 6 to 6.5 and neutralized, the non-glycerol organic fraction is removed, methanol is distilled off, then treated with an alkaline agent, and in step b) the water is removed by distillation to a content of 2% by weight, in step c) the pH of the mixture is adjusted to a value from 9 to 10 with an aqueous solution of an alkali hydroxide, preferably sodium hydroxide, and a sulphur-binding agent is introduced, which is an oxide of metal from the eighth group of the periodic table of elements, in the amount of 0.01% by weight, then in step d) distillation is performed and in step e) the glycerine after distillation is fed to a column containing an ion exchange resin, preferably a cation exchanger, then in step f) the mixture is subjected to steam stripping and bleaching in a system of adsorbers loaded with activated carbon and/or activated carbon with an additional adsorbent and filters. The invention also relates to glycerine purified by this method.

## Description

The present invention relates to a method of purifying glycerine resulting from transesterification of used cooking oils. The invention also relates to glycerine purified by this method.

Glycerol (propane-1,2,3-triol) is a polyol with a molecule containing 3 carbon atoms linked to hydroxyl groups. It is hygroscopic and dissolves in water. Producers manufacture a product called glycerine, which contains 60-99.7% by weight of glycerol. Crude glycerine is mainly obtained as a byproduct of fat hydrolysis during production of soaps and triglyceride transesterification, such as animal fats or vegetable oils, during production of FAME (fatty acid methyl esters). It is estimated that around 60-70% of the crude glycerine available on the global market comes from the production of biodiesel. Triglycerides react with methanol, and as a result of the reaction glycerol and esters (biodiesel) are formed in a molar ratio of 1:3 (in a weight ratio of about 1:10). The reaction occurs in the presence of an alkaline catalyst, usually potassium hydroxide or sodium hydroxide. The product of the transesterification process is a mixture of glycerol, methanol, water, inorganic salts, catalyst residues, free fatty acids (FFA), unreacted mono-, di- and triglycerides, methyl esters and many other organic substances, MONG (Matter Organic Non-Glycerol), in various proportions. The glycerol content is generally 60-80% by weight. The obtained mixture undergoes purification processes, which result in obtaining glycerine of types of quality. It is crude glycerine obtained after partial purification, mainly from catalyst residues, FFA and part of water, with a glycerol content of approx. 80% by weight, and refined glycerine obtained from the purification of crude glycerine, with a glycerol content of min. 99.5% by weight.

The Chinese patent application CN109721471A discloses a method in which methanol is added to crude glycerine, the mixture is acidified, centrifuged, the layers are separated and the layer with glycerine is used for further purification. In the next step, the pH is adjusted and the methanol is removed by distillation. The process is then carried out on resins (cation exchanger and anion exchanger) to obtain colourless glycerine. In the final stage, the glycerine is dehydrated.

The solution described in the U.S. patent US8545703B1 relates to the removal of sulphur in a glycerine purification process. For this purpose, a pre-treatment is realized by: filtration, separation of moisture from the filtrate to obtain a dry filtrate, distillation of the dry filtrate, and then distillation of the distillate obtained in the previous step. These last two steps can be repeated until the glycerine is of the desired degree of purity (including sulphur content).

Another Chinese patent application CN105418378A teaches that in order to obtain refined glycerine it must be purified and impurities separated. For this purpose, it is treated with acid (by adding a solvent and an inorganic acid solution) to obtain the desired pH of the solution (acidic), heated, stirred and the alkaline pH of the catalyst is neutralized. At the same time, the saponified substances are converted into fatty acids. It is further degummed with aluminium sulphate and FeCl₃ and neutralized with an alkali. The acid pH is neutralized by treatment with an alkali and FeCl₃ converted to Fe(OH)₃. The impurities are precipitated from this mixture, adsorbed and removed by filtration. In the next steps, the glycerine is concentrated, filtered and demineralised, as well as: purified (distillation), decolorized, rectified and ion exchanged.

In turn, the U.S. patent application US20110004031A1 describes a method of purifying glycerine. According to the referenced solution, in order to purify the glycerine in sequence: crude glycerine is acidified e.g. with sulphuric acid (with simultaneous addition of methanol), methanol is removed by distillation, the acidified solution is filtered and neutralized e.g. with calcium hydroxide, then the solution is filtered and ion exchanged on several columns for bleaching (activated carbon + alkaline ion exchange resin). The purpose of ion exchange is to remove salt ions that can affect the colour of the end product, e.g. iron salts. Next, the solution is deodorized on activated carbon and the glycerine is dehydrated.

The technical problem faced by the invention is to provide a method of purifying glycerine resulting from transesterification of used cooking oils (UCO) or vegetable oils, which would allow its purification to the quality of distilled glycerine (with a content of not less than 99.7% by weight) and the content of total sulphur, total nitrogen and total chlorides less than 2 ppm. Obtaining such purity and contents would allow its use in specialized chemical processes with high requirements as to the raw material quality, e.g. production of the highest purity propylene glycol.

The first subject of the invention is a method of purifying glycerine resulting from transesterification reaction of used cooking oils or vegetable oils, comprising the steps of:
a) removing non-glycerol organic fraction and methanol,
b) removing water,
c) removing sulphur-containing impurities,
d) glycerine distillation,
e) removing impurities containing nitrogen, chlorine and sulphur,
f) deodorization and bleaching,
characterized in that in step a) the feed is acidified with sulphuric acid (VI) to a pH of 6 to 6.5 and neutralized, the non-glycerol organic fraction is removed, methanol is distilled off, then treated with an alkaline agent, and in step b) the water is removed by distillation to a content of 2% by weight, in step c) the pH of the mixture is adjusted to a value from 9 to 10 with an aqueous solution of an alkali hydroxide, preferably sodium hydroxide, and a sulphur-binding agent is introduced, which is an oxide of metal from the eighth group of the periodic table of elements, in the amount of 0.01% by weight, then in step d) distillation is performed and in step e) the glycerine after distillation is fed to a column containing an ion exchange resin, preferably a cation exchanger, then in step f) the mixture is subjected to steam stripping and bleaching in a system of adsorbers loaded with activated carbon and/or activated carbon with an additional adsorbent and filters.

In a preferred embodiment of the invention, the glycerine to be purified contains up to 5% by weight of used cooking oils.

In a further preferred embodiment of the invention, the glycerine to be purified contains no more than 35% by weight of water, preferably not more than 30% by weight, even more preferably not more than 26% by weight.

In a further preferred embodiment of the invention, the alkaline agent is selected from the group containing: Ba(OH)₂·8H₂O, Ca(OH)₂, CaO or MgO.

In another preferred embodiment of the invention, the pH of the reaction mixture during reaction with the alkaline agent is maintained between 3 and 4.

In yet another preferred embodiment of the invention, the reaction with the alkaline agent is carried out at a temperature of 60°C to 80°C.

In a still further preferred embodiment of the invention, the pH in step a) is adjusted with an alkali metal hydroxide, preferably lithium, sodium or potassium hydroxide.

In yet another preferred embodiment of the invention, the non-glycerol organic fraction is removed by centrifugation prior to methanol removal.

In a preferred embodiment of the invention, the non-glycerol organic fraction and the alkaline reaction products are removed by filtration after methanol removal.

In a preferred embodiment of the invention, the methanol in step a) is removed by rectification.

In another preferred embodiment of the invention, the water in step b) is removed in two successive substeps.

In a preferred embodiment of the invention, in the first substep water is removed to a content of 10% by weight of the mixture and the mixture is transferred to the second substep.

In a further preferred embodiment of the invention, in the second substep water is distilled off to a content of not more than 2% by weight.

In yet another preferred embodiment of the invention, the sulphur-binding agent is iron (III) oxide.

In a preferred embodiment of the invention, the additional adsorbent is silicon oxide or bleaching clay.

In another preferred embodiment of the invention, the additional adsorbent is 1% by weight of the bed.

In another preferred embodiment of the invention, the neutralisation in step a) is carried out with an aqueous solution of alkali hydroxides at a concentration of 50% by weight, preferably sodium hydroxide or potassium hydroxide.

The second subject of the invention is purified glycerine resulting from transesterification reaction of used cooking oils or vegetable oils, characterized in that it contains glycerine in an amount of 98% to 99.8% by weight and water in an amount of 0.2% to 2% by weight of the composition, being substantially free of impurities, having a total sulphur content of not more than 2 mg/kg, a total nitrogen content of not more than 2 mg/kg, a total chlorine content of not more than 2 mg/kg.

The purification of glycerine resulting from transesterification of used cooking oils (UCO) or vegetable oils is carried out in several steps. These types of oils can be contaminated with proteins, salts and fats. After the purification process, the oil should meet the standard requirements. According to PN-EN ISO 663, the content of insoluble impurities in the raw materials being the feed is in the range of 0.1-0.4%; the content of free fatty acids according to PN-EN ISO 660 is in the range of 0.2-4.0% (rapeseed oil <0.5%), and the content of water and volatile substances according to PN-EN ISO 662 is in the range of 0.1-1.0%, as well as the content of sulphur is in the range of 5-25 mg/kg (rapeseed oil <10 mg/kg).

The feed of the raw material with a pH of about 10-14 is acidified in such a way that the pH at the output is 6-6.5, and then neutralized with a 50% aqueous solution of NaOH or KOH. The feed may be a mixture of glycerol, water, methanol and a metal hydroxide, e.g. sodium or potassium hydroxide. Hydroxides are used as catalysts in the transesterification process. This process leads to breaking down soaps dissolved in glycerine into fatty acids and neutralization of potassium hydroxide with sulphuric acid (96% by weight) and forming the corresponding salts, which are separated on the decanters. In the next step, methanol is separated from glycerol in the rectification column. Glycerine is preheated before feeding to the column and then fed to the column, where the rectification process takes place. Methanol with a purity of min. 99.8% is received and condensed and partly used as reflux. After this step, the process mixture (crude glycerine) contains 15-35% by weight of water. After methanol removal, the water-insoluble non-glycerine organic fraction (MONG - Matter Organic Non-Glycerol), consisting of a mixture of esters, free fatty acids, fatty acid salts and soaps, is centrifuged. For an effective purification process of the crude glycerine, maximum removal is essential. It is preferable to keep the crude glycerine pH at a level of 3-4. Further, an alkaline agent is added to the process mixture. The alkaline agent may be hydroxides or oxides of metals of the second group of the periodic table. Basically these are barium hydroxide (Ba(OH)₂), calcium hydroxide Ca(OH)₂, calcium oxide (CaO) or magnesium oxide (MgO). Introduction of the alkaline agent leads to pH increase to 5-6 and reaction with sulphate ions contained in the glycerine. The reaction with the alkaline agent is carried out at the temperature of 60-80°C and for 1-2 hours, with continuous stirring. The reaction with the alkaline agent aims to further separate the non-glycerol organic fraction, which was not removed after acidification, and inorganic residues from the mixture of components. Removal of these impurities is carried out on filter presses. After their removal, glycerine is dehydrated to the level of about 2%. It is a two-stage process. Water is distilled off from glycerine to a content of about 10% by weight in a distillation column. This column operates in a vacuum system. Glycerine with a water content of about 10% is then directed to a second column for drying to a water content of not more than 2% by weight. In the next process node, after the drying node, the pH is adjusted to a level of 9-10 by adding 50% aqueous solution of sodium hydroxide. Then, an oxide of metal from the eighth group of the periodic table of elements is introduced into the mixture thus prepared. The oxide is the sulphur-binding agent in the process mixture. Particularly preferred effects are achieved by adding iron (III) oxide. It is introduced in an amount of 0.01% by weight of the mixture subjected to purification on this node. It allows to reduce the content of total sulphur (organic and mineral) in the distilled glycerine from 3-4 mg/kg to a level below 2 mg/kg. The process of adjusting the pH to the level of 9-10 by adding an aqueous solution of NaOH and the reaction with iron (III) oxide are carried out in one reactor. After sulphur removal and the pH adjustment, glycerine is distilled. The feedstock is directed to the vacuum column distillation process. As a result of the process, distilled glycerine is obtained, the remaining water and volatile components are evaporated. The distillation residue is the MONG which is collected in an IBC (Intermediate Bulk Container) tank. The distilled glycerine is in the next step purified on an ion exchange resin, preferably on the cation exchanger Amberlite^{™} IR120, H+ form in the form of a gel. This leads to a reduction in total nitrogen (organic and mineral) in the distilled glycerine from 17 mg/kg to 1.2 mg/kg, total chlorides (organic and inorganic chlorides) from 2-6 mg/kg to <1 mg/kg, as well as the sulphur content from 3.6 mg/kg to 1.4 mg/kg. The glycerine is then deodorized and bleached. The deodorization of glycerine is performed in a column in the stripping process. This process is designed to evaporate the substances responsible for the specific smell of glycerine. On the other hand, the bleaching system consists of three adsorbers with a fixed bed of activated carbon and filters. Adsorbers work in series in any configuration. When the carbon is spent, the bed is replaced. The modification of the unit process will consist of adding an additional adsorbent, e.g. SiO₂, to the activated carbon.

The solution of the invention is characterized by a number of advantages. First of all, it enables the utilization of glycerine of plant and animal origin, contaminated in a specific way, including UCO. This will allow to sell this type of glycerine to chemical synthesis processes. Moreover, obtaining such glycerine of high purity (>99.7% by weight) and low content of sulphur, nitrogen and chlorides, in relation to the invention being the sum of their organic and inorganic forms, allows for its use in specialized chemical processes with high requirements as to raw material quality, e.g. production of the highest purity propylene glycol. The high profitability of the solution improves the economic efficiency indicators of producers of methyl esters of higher fatty acids.

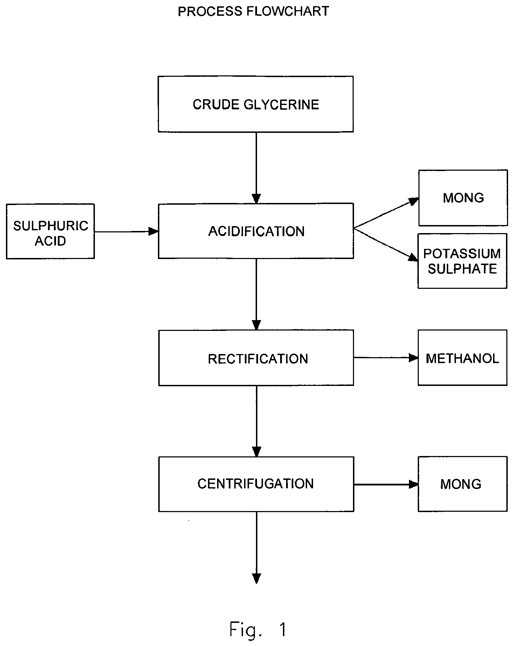

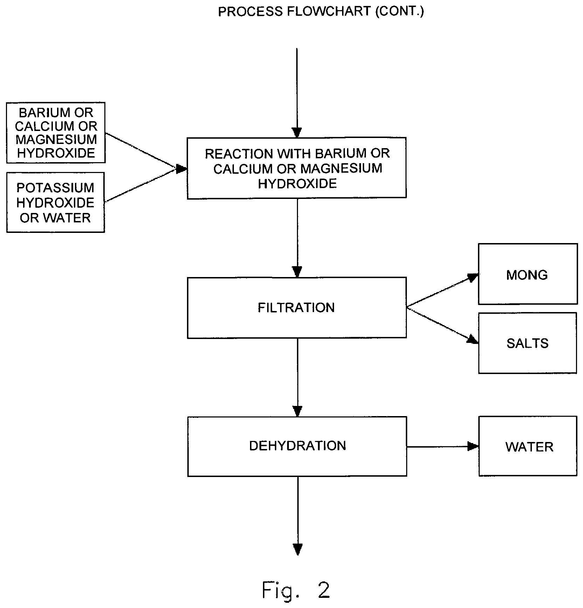

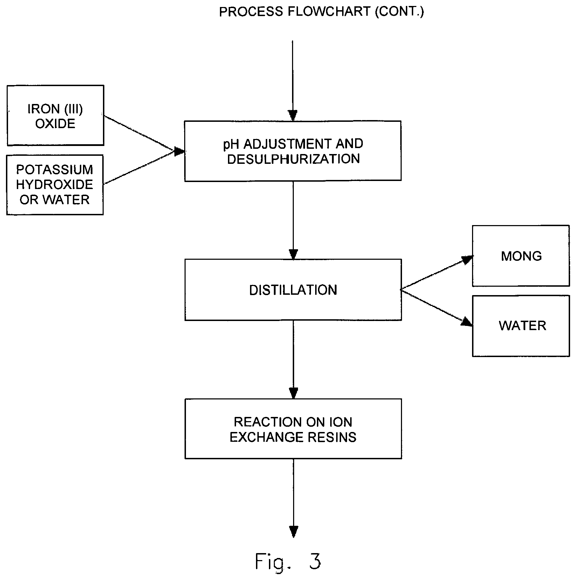

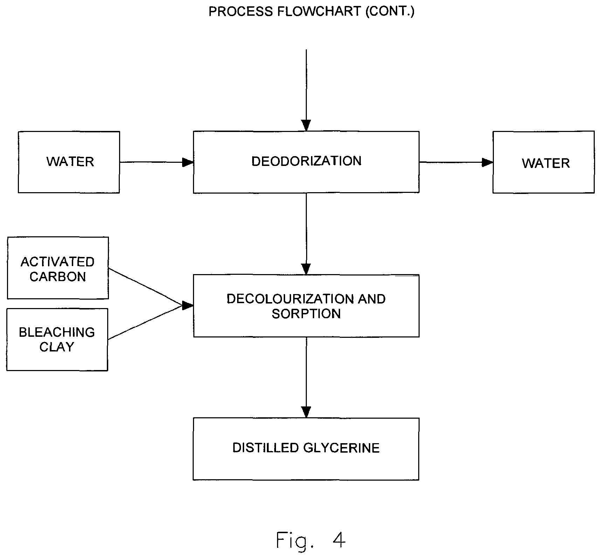

The drawings (Figs. 1-4) show a process flowchart for the purification of glycerine resulting from transesterification of Used Cooking Oils (UCO) or vegetable oils, e.g. rapeseed oil. Figures 2-4 are a continuation of the process shown in figure 1. In the embodiments of the invention (examples 1-6), the purification of the above-mentioned glycerine for different raw material compositions is illustrated.

### Example 1

The process of purifying glycerine resulting from UCO transesterification was carried out. For this purpose, crude glycerine sample was first acidified (allowing the breakdown of soaps dissolved therein into fatty acids) to pH 6-6.5, and then neutralized with 50% aqueous NaOH or KOH solution. Secondly, in order to separate the residual methanol, it was directed to the rectification process. Next, the separation of the so-called MONG (Matter Organic Non-Glycerol) was performed on a centrifuge. The glycerine was then directed to a reactor to react the glycerine with 0.5% Ba(OH)₂·8H₂O at 60°C for 60 minutes. Ca(OH)₂ can also be used instead of Ba(OH)₂·8H₂O. The conditions of introducing into the reaction mixture do not differ, while the amount of dosed Ca(OH)₂ (0.2-1%) is different from Ba(OH)₂·8H₂O (0.5-3%). The glycerine of the reaction was then directed to the filtration process. Then it was subjected to the process of water evaporation, as a result of which it contained 1.7 mg/kg of sulphur and 17 mg/kg of nitrogen. Further, the reaction of binding the sulphur contained in the glycerine was carried out by contacting it with 0.01% addition of iron oxide Fe₂O₃. The feed stream was then directed to a distillation column whereby residual water and volatile components were evaporated. Then, the process of contacting the distilled glycerine with a cationic ion exchange resin was carried out in order to reduce the content of undesirable components containing elements such as S, N, CI. In order to deodorize the glycerine, the process of steam stripping was used, followed by sorption on activated carbon. Finally, a product with the following quality parameters is obtained:

| **Property** | **Value** |
|---|---|
| Density at 15°C, kg/m³ | 1262 |
| Free glycerol content, GC meth., % | 99.8 |
| pH | 5.53 |
| Sulphur content, mg/kg | 1.3 |
| Nitrogen content, mg/kg | <1.0 |
| Chlorine content, mg/kg | <1.0 |

### Example 2

The process of purifying glycerine resulting from transesterification of 95% rapeseed oil with the addition of 5% UCO (Used Cooking Oils) was carried out. For this purpose, crude glycerine sample was first acidified (allowing the breakdown of soaps dissolved therein into fatty acids), and then neutralized as in Example 1. Secondly, in order to separate the residual methanol, it was directed to the rectification process. Next, the separation of the so-called MONG (Matter Organic Non-Glycerol) was performed on a centrifuge. The glycerine was then directed to a reactor to react the glycerine with 0.1% CaO at 60°C for 60 minutes. MgO can also be used instead of CaO. The glycerine of the reaction was then directed to the filtration process. Then it was subjected to the process of water evaporation, as a result of which it contained 3.2 mg/kg of sulphur and 3.6 mg/kg of nitrogen. Further, the reaction of binding the sulphur contained in the glycerine was carried out by contacting it with 0.01 % addition of iron oxide Fe₂O₃. The feed stream was then directed to a distillation column whereby residual water and volatile components were evaporated. Then, the process of contacting the distilled glycerine with a cationic ion exchange resin was carried out in order to reduce the content of undesirable components containing elements such as S, N, CI. In order to deodorize the glycerine, the process of steam stripping was used, followed by sorption on activated carbon. Finally, a product with the following quality parameters is obtained:

| **Property** | **Value** |
|---|---|
| Density at 15°C, kg/m³ | 1260 |
| Free glycerol content, GC meth., % | 98.5 |
| pH | 3.2 |
| Sulphur content, mg/kg | 1.4 |
| Nitrogen content, mg/kg | <1.0 |
| Chlorine content, mg/kg | <1.0 |

### Example 3

The process of purifying technical glycerine resulting from transesterification of rapeseed oil and 5% UCO, containing approx. 13% water, was carried out. For this purpose, crude glycerine sample was first acidified (allowing the breakdown of soaps dissolved therein into fatty acids), and then neutralized as in Example 1. Secondly, in order to separate the residual methanol, it was directed to the rectification process. Next, the separation of the so-called MONG (Matter Organic Non-Glycerol) was performed on a centrifuge. The glycerine was then directed to a reactor to react the glycerine with 0.1% CaO at 60°C for 90 minutes. The glycerine of the reaction was then directed to the filtration process. Then it was subjected to the process of water evaporation. Further, the reaction of binding the sulphur contained in the glycerine was carried out by contacting it with 0.01% addition of iron oxide Fe₂O₃. The feed stream was then directed to a distillation column whereby residual water and volatile components were evaporated. Then, the process of contacting the distilled glycerine with a cationic ion exchange resin was carried out in order to reduce the content of undesirable components containing elements such as S, N, CI. In order to deodorize the glycerine, the process of steam stripping was used, followed by sorption on activated carbon. Finally, a product with the following quality parameters is obtained:

| **Property** | **Value** |
|---|---|
| Density at 15°C, kg/m³ | 1260.7 |
| Free glycerol content, GC meth., % | 98.5 |
| pH | 4.09 |
| Sulphur content, mg/kg | 1.9 |
| Nitrogen content, mg/kg | 1.5 |
| Chlorine content, mg/kg | <1.0 |

### Example 4

The process of purifying glycerine resulting from UCO (Used Cooking Oils) transesterification was carried out. For this purpose, crude glycerine sample was first acidified (allowing the breakdown of soaps dissolved therein into fatty acids), and then neutralized as in Example 1. Secondly, in order to separate the residual methanol, it was directed to the rectification process. Next, the separation of the so-called MONG (Matter Organic Non-Glycerol) was performed on a centrifuge. The glycerine was then directed to a reactor to react the glycerine with 0.5% Ba(OH)₂·8H₂O at 60°C for 90 minutes. The glycerine of the reaction was then directed to the filtration process. Then it was subjected to the process of water evaporation, as a result of which it contained 1.7 mg/kg of sulphur and 17 mg/kg of nitrogen. Further, the reaction of binding the sulphur contained in the glycerine was carried out by contacting it with 0.01% addition of iron oxide Fe₂O₃. The feed stream was then directed to a distillation column whereby residual water and volatile components were evaporated. Then, the process of contacting the distilled glycerine with a cationic ion exchange resin was carried out in order to reduce the content of undesirable components containing elements such as S, N, Cl. In order to deodorize the glycerine, the process of steam stripping was used, followed by sorption on activated carbon. Finally, a product with the following quality parameters is obtained:

| **Property** | **Value** |
|---|---|
| Density at 15°C, kg/m³ | 1263 |
| Free glycerol content, GC meth., % | 99.8 |
| pH | 5.5 |
| Sulphur content, mg/kg | 1.3 |
| Nitrogen content, mg/kg | <1.0 |
| Chlorine content, mg/kg | <1.0 |

### Example 5

The process of purifying technical glycerine resulting from transesterification of rapeseed oil and 5% UCO, containing approx. 18% water, was carried out. For this purpose, crude glycerine sample was first acidified (allowing the breakdown of soaps dissolved therein into fatty acids), and then neutralized as in Example 1. Secondly, in order to separate the residual methanol, it was directed to the rectification process. Next, the separation of the so-called MONG (Matter Organic Non-Glycerol) was performed on a centrifuge. The glycerine was then directed to a reactor to react the glycerine with 0.1% CaO at 60°C for 90 minutes. The glycerine of the reaction was then directed to the filtration process. Then it was subjected to the process of water evaporation. Further, the reaction of binding the sulphur contained in the glycerine was carried out by contacting it with 0.01% addition of iron oxide Fe₂O₃. The feed stream was then directed to a distillation column whereby residual water and volatile components were evaporated. Then, the process of contacting the distilled glycerine with a cationic ion exchange resin was carried out in order to reduce the content of undesirable components containing elements such as S, N, Cl. In order to deodorize the glycerine, the process of steam stripping was used, followed by sorption on activated carbon with 1% addition of SiO₂. Finally, a product with the following quality parameters is obtained:

| **Property** | **Value** |
|---|---|
| Density at 15°C, kg/m³ | 1262.4 |
| Free glycerol content, GC meth., % | 99.4 |
| pH | 5.2 |
| Sulphur content, mg/kg | 1.8 |
| Nitrogen content, mg/kg | 1.2 |
| Chlorine content, mg/kg | <1.0 |

## Claims

1. A method of purifying glycerine resulting from transesterification reaction of used cooking oils or vegetable oils, comprising the steps of:
a) removing non-glycerol organic fraction and methanol,
b) removing water,
c) removing sulphur-containing impurities,
d) glycerine distillation,
e) removing impurities containing nitrogen, chlorine and sulphur,
f) deodorization and bleaching,
**characterized in that** in step a) the feed is acidified with sulphuric acid (VI) to a pH of 6 to 6.5 and neutralized, the non-glycerol organic fraction is removed, methanol is distilled off, then treated with an alkaline agent, and in step b) the water is removed by distillation to a content of 2% by weight, in step c) the pH of the mixture is adjusted to a value from 9 to 10 with an aqueous solution of an alkali hydroxide, preferably sodium hydroxide, and a sulphur-binding agent is introduced, which is an oxide of metal from the eighth group of the periodic table of elements, in the amount of 0.01% by weight, then in step d) distillation is performed and in step e) the glycerine after distillation is fed to a column containing an ion exchange resin, preferably a cation exchanger, then in step f) the mixture is subjected to steam stripping and bleaching in a system of adsorbers loaded with activated carbon and/or activated carbon with an additional adsorbent and filters.

2. The method of claim 1, **characterized in that** the glycerine to be purified contains up to 5% by weight of used cooking oils.

3. The method of claim 1, **characterized in that** the glycerine to be purified contains no more than 35% by weight of water, preferably not more than 30% by weight, even more preferably not more than 26% by weight.

4. The method of claim 1, **characterized in that** the alkaline agent is selected from the group containing: Ba(OH)₂·8H₂O, Ca(OH)₂, CaO or MgO.

5. The method of claim 1 or 4, **characterized in that** the pH of the reaction mixture during reaction with the alkaline agent is maintained between 3 and 4.

6. The method of claim 1, 4 or 5, **characterized in that** the reaction with the alkaline agent is carried out at a temperature of 60°C to 80°C.

7. The method of claim 1, **characterized in that** the pH in step a) is adjusted with an alkali metal hydroxide, preferably lithium, sodium or potassium hydroxide.

8. The method of claim 1, **characterized in that** the non-glycerol organic fraction is removed by centrifugation prior to methanol removal.

9. The method of claim 1, **characterized in that** the non-glycerol organic fraction and alkaline reaction products are removed by filtration after methanol removal.

10. The method of claim 1, **characterized in that** the methanol in step a) is removed by rectification.

11. The method of claim 1, **characterized in that** the water in step b) is removed in two successive substeps, preferably in the first substep water is removed to a content of 10% by weight of the mixture and the mixture is transferred to the second substep, wherein with particular preference in the second substep water is distilled off to a content of not more than 2% by weight.

12. The method of claim 1, **characterized in that** the sulphur-binding agent is iron (III) oxide.

13. The method of claim 1, **characterized in that** the additional adsorbent is silicon oxide or bleaching clay.

14. The method of claim 1 or 13, **characterized in that** the additional adsorbent is 1% by weight of the bed.

15. The method of claim 1, **characterized in that** the neutralisation in step a) is with an aqueous solution of alkali hydroxides at a concentration of 50% by weight, preferably sodium hydroxide or potassium hydroxide.

16. Purified glycerine resulting from transesterification reaction of used cooking oils or vegetable oils, **characterized in that** it contains glycerine in an amount of 98% to 99.8% by weight and water in an amount of 0.2% to 2% by weight of the composition, being substantially free of impurities, having a total sulphur content of not more than 2 mg/kg, a total nitrogen content of not more than 2 mg/kg, a total chlorine content of not more than 2 mg/kg.
